(19) Europäisches Patentamt
European Patent Office
Office européen des brevets

(11) Veröffentlichungsnummer: **0 199 974**
**A2**

(12) **EUROPÄISCHE PATENTANMELDUNG**

(21) Anmeldenummer: **86103881.8**

(51) Int. Cl.[4]: **C07D 239/06** , A01N 43/54

(22) Anmeldetag: **21.03.86**

(30) Priorität: **02.04.85 JP 68551/85**

(43) Veröffentlichungstag der Anmeldung:
**10.12.86 Patentblatt 86/45**

(84) Benannte Vertragsstaaten:
**AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Shiokawa, Kozo**
**210-6, Shukugawara Tama-ku**
**Kawasaki-shi Kanagawa-ken(JP)**
Erfinder: **Tsuboi, Shinichi**
**3-26-1, Hirayama**
**Hino-shi Tokyo(JP)**
Erfinder: **Kagabu, Shinzo**
**432-131-105, Terada-machi**
**Hachioji-shi Tokyo(JP)**
Erfinder: **Moriya, Koichi**
**39-15, Namiki-cho**
**Hachioji-shi Tokyo(JP)**

(74) Vertreter: **Ernst, Hilmar, Dr. et al**
**Bayer AG Konzernverwaltung RP**
**Patentabteilung**
**D-5090 Leverkusen, Bayerwerk(DE)**

(54) **Nitromethylen-tetrahydropyrimidine.**

(57) Die vorliegende Erfindung betrifft neue Nitromethylen-tetrahydropyrimidin-Derivate der Formel (I)

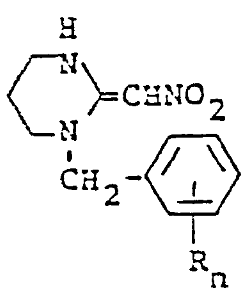

(I)

EP 0 199 974 A2

in der

R eine Alkyl-Gruppe, eine Alkoxy-Gruppe, eine Alkylthio-Gruppe, eine Nitro-Gruppe, eine Cyano-Gruppe, eine halogen-substituierte Alkyl-Gruppe, eine halogen-substituierte Alkoxy-Gruppe, eine halogen-substituierte Alkylthio-Gruppe, eine Amino-Gruppe, eine Dialkylamino-Gruppe, eine Acylamino-Gruppe, eine Carboxyl-Gruppe, eine Alkoxycarbonyl-Gruppe, eine Alkylsulfinyl-Gruppe, eine Alkylsulfonyl-Gruppe oder ein Halogen-Atom bezeichnet und

n 1, 2 oder 3 bezeichnet, jedoch mit der Maßgabe, daß, wenn n für 1 steht, R nicht ein Halogen-Atom bezeichnen darf,

Verfahren zur Herstellung der Verbindungen (I) sowie die Verwendung der neuen Verbindungen als Pestizide, insbesondere als Insektizide.

Nitromethylen-tetrahydropyrimidine

Die vorliegende Erfindung betrifft neue Nitromethylen-tetrahydropyrimidin-Derivate, ein Verfahren zu ihrer Herstellung sowie die Verwendung dieser Verbindungen als Pestizide, insbesondere als Insektizide.

Es ist bereits bekannt, daß Nitromethylenimidazolidin-Derivate insektizide Aktivitäten besitzen (siehe die DE-OS 27 32 660).

Gemäß der vorliegenden Erfindung wurden neue Nitromethylen-tetrahydropyrimidine der Formel (I)

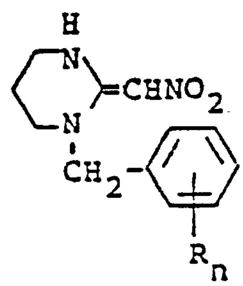

(I)

gefunden.

In der Formel bezeichnet

R eine Alkyl-Gruppe, eine Alkoxy-Gruppe, eine Alkylthio-Gruppe, eine Nitro-Gruppe, eine Cyano-Gruppe, eine halogen-substituierte Alkyl-Gruppe, eine halogen-substituierte Alkoxy-Gruppe, eine halogen-substituierte Alkylthio-Gruppe, eine Amino-Gruppe, eine Dialkylamino-Gruppe, eine Acylamino-Gruppe, eine Carboxyl-Gruppe, eine Alkoxycarbonyl-Gruppe, eine Alkylsulfinyl-Gruppe, eine Alkylsulfonyl-Gruppe oder ein Halogen-Atom, und

n bezeichnet 1, 2 oder 3, jedoch mit der Maßgabe, daß, wenn n für 1 steht, R nicht ein Halogen-Atom bezeichnen darf.

Die Verbindungen der Formel (I) werden erhalten, wenn (a) die Verbindungen der Formel (II)

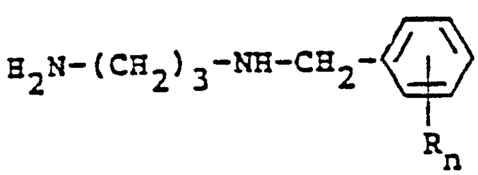

(II)

in der R und n die oben angegebenen Bedeutungen haben, mit Verbindungen der Formel

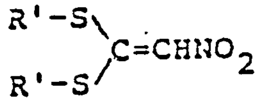

(III)

in der R' eine Niederalkyl-Gruppe oder eine Benzyl-Gruppe bezeichnet, oder die beiden Gruppen R' zusammen genommen eine niedere Alkylen-Gruppe mit wenigstens 2 Kohlenstoff-Atomen bezeichnen oder zusammen mit den benachbarten Schwefel-Atomen einen Ring bilden können, gegebenenfalls in Gegenwart inerter Lösungsmittel umgesetzt werden, oder

(b) die Verbindung der nachstehenden Formel

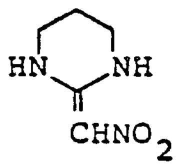

mit einer der Verbindungen der Formel (IV)

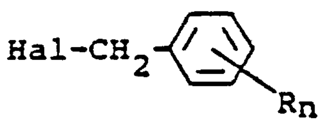

(IV)

in der R und n die im Vorstehenden angegebenen Bedeutungen haben und Hal ein Halogen-Atom ist, umgesetzt wird, gegebenenfalls in Gegenwart inerter Lösungsmittel und/oder säurebindender Mittel.

Die neuen Nitromethylen-tetrahydropyrimidine der Formel (I) zeigen starke insektizide Eigenschaften.

Überraschenderweise zeigen die Nitromethylen-tetrahydropyrimidine gemäß der vorliegenden Erfindung eine wesentlich größere insektizide Wirksamkeit als analoge Verbindungen, die aus dem bisherigen Stand der Technik bekannt sind.

Außerdem wurde gefunden, daß die Verbindungen der vorliegenden Erfindung eine bemerkenswerte Bekämpfungswirkung gegenüber stechenden und saugenden Insekten zeigen, wie sie typisch durch Insekten der Gattung Hemiptera repräsentiert werden, etwa Blattläuse, Wanzen, Heuschrecken und dergleichen.

Darüber hinaus zeigen die Verbindungen der vorliegenden Erfindung besonders gute Bekämpfungseffekte, wenn sie mittels Wasseroberflächenbehandlung zur Anwendung gebracht werden, und sind in bezug auf Rückstandswirkungen in Wasser der Verbindung der nachstehenden Formel (A-1) ausgesprochen überlegen, die den Verbindungen der vorliegenden Erfindung am ähnlichsten ist (vgl. die DE-OS 27 32 660).

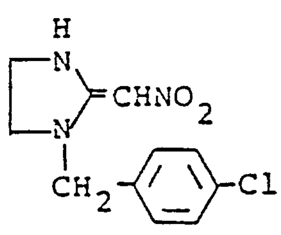

(A-1)

Von den erfindungsgemäßen Verbindungen der Formel (I) sind bevorzugte Verbindungen diejenigen, in denen

R eine Alkyl-Gruppe mit 1 bis 4 Kohlenstoff-Atomen, eine Alkoxy-Gruppe mit 1 bis 4 Kohlenstoff-Atomen, eine Alkylthio-Gruppe mit 1 bis 4 Kohlenstoff-Atomen, eine Nitro-Gruppe, eine Cyano-Gruppe, eine halogen-substituierte Alkyl-Gruppe mit 1 bis 4 Kohlenstoff-Atomen, eine halogen-substituierte Alkoxy-Gruppe mit 1 bis 4 Kohlenstoff-Atomen, eine halogen-substituierte Alkylthio-Gruppe mit 1 bis 4 Kohlenstoff-Atomen, eine Amino-Gruppe, eine Dialkylamino-Gruppe mit 1 bis 4 Kohlenstoff-Atomen in jeder Alkyl-Struktureinheit, eine Acetylamino-Gruppe, eine Carboxyl-Gruppe, eine Alkoxycarbonyl-Gruppe mit 2 bis 5 Kohlenstoff-Atomen, eine Alkylsulfinyl-Gruppe mit 1 bis 4 Kohlenstoff-Atomen oder ein Halogen-Atom ist, und

n 1 oder 2 ist, jedoch mit der Maßgabe, daß, wenn n 1 ist, R nicht ein Halogen-Atom bezeichnet.

Verschiedene besonders bevorzugte Verbindungen der Formel (I) sind diejenigen, in denen R Methyl, Ethyl, Methoxy, Ethoxy, Methylthio, Ethylthio, Nitro, Cyano, Trifluormethyl, Trifluormethoxy, Trifluormethylthio, 2,2,2-Trifluoroethylthio, Amino, Dimethylamino, Acetylamino, Carboxy, Methoxycarbonyl, Methylsulfinyl, Methylsulfonyl, Fluor , Chlor oder Brom bezeichnet und n 1 oder 2 ist, jedoch mit der Maßgabe, daß, wenn n 1 ist, R nicht Fluor , Chlor oder Brom bezeichnet.

Spezielle Beispiele der Verbindungen der Formel (I) gemäß der vorliegenden Erfindung seien besonders erwähnt:

1-(4-Trifluormethylbenzyl)-2-(nitromethylen)-tetrahydropyrimidin,

1-(4-Tolylmethyl)-2-(nitromethylen)-tetrahydropyrimidin,

1-(4-Nitrobenzyl)-2-(nitromethylen)-tetrahydropyrimidin und

1-(4-Methoxybenzyl)-2-(nitromethylen)-tetrahydropyrimidin.

Die Verbindungen der Formel (I) können mittels des folgenden Verfahrens hergestellt werden.

Verfahren a)

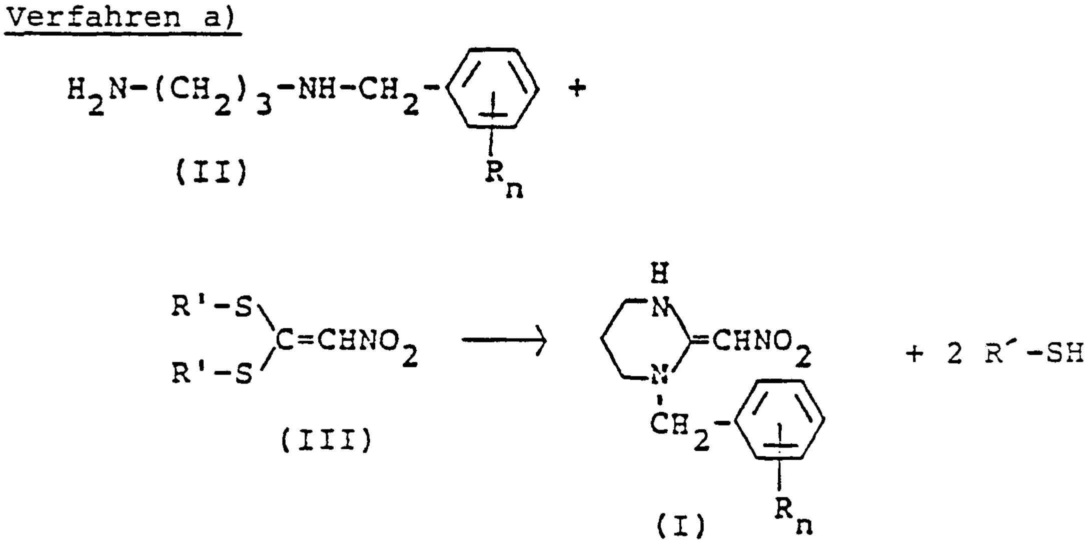

(II)

(III)

(I)

(In den Formeln haben R, n und R' die oben angegebenen Bedeutungen).

Das vorstehende Verfahren wird durch das folgende Reaktionsschema dargestellt, sofern N-(4-Trifluormethylbenzyl)trimethylendiamin und 1-Nitro-2,2-bis(methylthio)ethylen als Ausgangsstoffe eingesetzt werden:

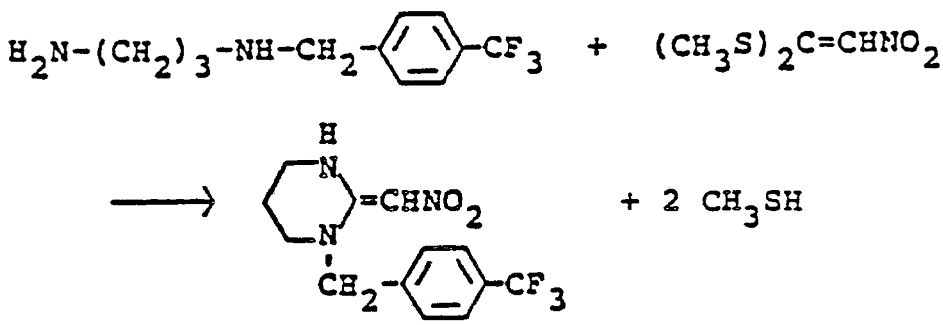

In dem vorstehenden Verfahren a) bezeichnet die Verbindung (II) als Ausgangsmaterial Verbindungen auf der Grundlage der vorstehenden Definitionen für R und n, und R und n haben vorzugsweise die oben angegebenen Bedeutungen.

Die erfindungsgemäß einzusetzenden Verbindungen der Formel (II) sind nicht bekannt, und als Beispiele seien erwähnt:

N-(4-Methylbenzyl)trimethylendiamin,

N-(4-Ethylbenzyl)trimethylendiamin,

N-(2-Methoxybenzyl)trimethylendiamin,

N-(4-Methoxybenzyl)trimethylendiamin,

N-(4-Methylthiobenzyl)trimethylendiamin,

N-(3-Nitrobenzyl)trimethylendiamin,

N-(4-Nitrobenzyl)trimethylendiamin,

N-(3-Aminobenzyl)trimethylendiamin,

N-(3-Dimethylaminobenzyl)trimethylendiamin,

N-(4-Dimethylaminobenzyl)trimethylendiamin,

N-(3-Trifluormethylbenzyl)trimethylendiamin,

N-(4-Trifluormethylthiobenzyl)trimethylendiamin,

N-(4-Cyanobenzyl)trimethylendiamin,

N-(3-Cyanobenzyl)trimethylendiamin,

N-(4-Trifluormethylbenzyl)trimethylendiamin,

N-(4-Trifluormethoxybenzyl)trimethylendiamin,

N-(4-Acetamidobenzyl)trimethylendiamin,

N-(3-Acetamidobenzyl)trimethylendiamin,

N-(4-Carboxybenzyl)trimethylendiamin,

N-(4-Methoxycarbonylbenzyl)trimethylendiamin,

N-(4-Methylsulfinylbenzyl)trimethylendiamin,

N-(4-Methylsulfonylbenzyl)trimethylendiamin,

N-(3,4-Dichlorbenzyl)trimethylendiamin,

N-(4-Aminobenzyl)trimethylendiamin,

N-[4-(1,1,2,2-Tetrafluorethoxy)benzyl]-trimethylendiamin,

N-(4-Difluormethoxybenzyl)trimethylendiamin,

N-(4-Ethoxybenzyl)trimethylendiamin,

N-[-4-(2,2,2-Trifluorethylthio)benzyl]-trimethylendiamin,

N-(3-Methylbenzyl)trimethylendiamin,

N-(4-Fluormethylbenzyl)trimethylendiamin und

N-[4-(2,2,2-Trifluorethoxy)benzyl]trimethylendiamin.

Die Verbindungen der Formel (II) können beispielsweise mittels des folgenden bekannten Verfahrens hergestellt werden.

Verfahren c)

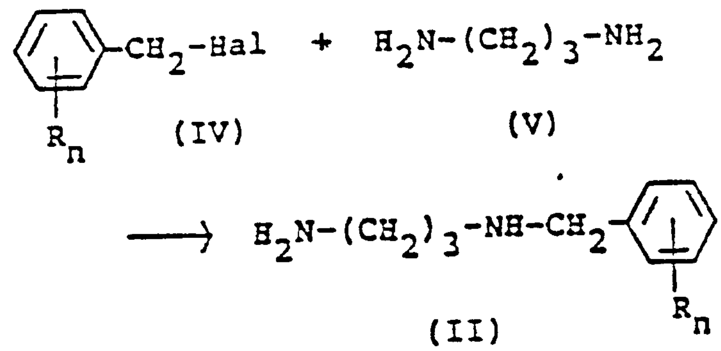

(IV) (V) (II)

(In den Formeln haben R und n die oben angegebenen Bedeutungen, und Hal bezeichnet ein Halogen-Atom).

Die meisten der Ausgangs-Verbindungen der Formel (IV) in dem Verfahren c) sind bekannte Verbindungen und können leicht mit Hilfe bekannter Verfahrensweisen hergestellt werden. Die Verbindungen der Formel (V) waren ebenfalls vor dem Prioritätszeitpunkt der vorliegenden Anmeldung bekannt und sind in der DE-OS 25 14 402 beschrieben. Das Verfahren c) kann in einfacher Weise entsprechend dem in der DE-OS 27 32 660 beschriebenen Verfahren durchgeführt werden.

Die als Ausgangsstoffe in dem Verfahren a) eingesetzten Verbindungen der Formel (III) sind bekannte Verbindungen und beispielsweise in Chem. Ber. 100, Seiten 591-604, beschrieben. Zu speziellen Beispielen zählen 1-Nitro-2,2-bis-(methylthio)ethylen, 1-Nitro-2,2-bis(ethylthio)-ethylen, 1-Nitro-2,2-bis(benzylthio)ethylen und 2-Nitromethylen-1,3-dithiolan.

In dem Verfahren a) können sämtliche inerten organischen Lösungsmittel als geeignete Verdünnungsmittel verwendet werden.

Beispiele für solche Verdünnungsmittel umfassen Wasser, aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Das vorstehende Verfahren kann in einem weiten Temperaturbereich durchgeführt werden. Beispielsweise kann es bei einer Temperatur zwischen etwa -20°C und dem Siedepunkt der Mischung, vorzugsweise zwischen etwa 0°C und etwa 100°C, durchgeführt werden. Die Reaktion wird vorzugsweise unter normalem Atmosphärendruck durchgeführt, jedoch ist es auch möglich, unter erhöhtem oder vermindertem Druck zu arbeiten.

In dem vorstehenden Verfahren können die gewünschten neuen Verbindungen der Formel (I) beispielsweise dadurch erhalten werden, daß 1 mol der Verbindung der Formel (II) und 1 bis etwa 1,2 mol, vorzugsweise 1 bis etwa 1,1 mol, der Verbindung der Formel (III) in einem inerten Lösungsmittel wie einem Alkohol (z.B. Methanol oder Ethanol) unter Rückfluß erhitzt werden.

Die Verbindungen der Formel (I) können auch mittels des folgenden Verfahrens b) hergestellt werden.

Verfahren b)

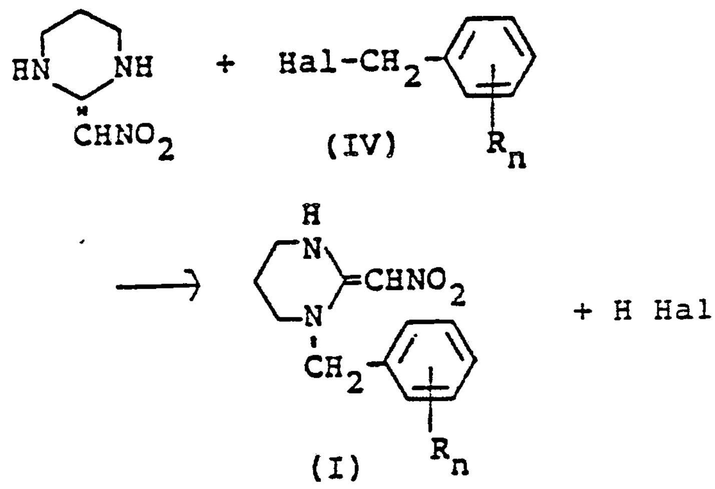

(In den Formeln haben R, n und Hal die oben angegebenen Bedeutungen).

In dem vorstehenden Verfahren b) ist 2-Nitromethylentetrahydropyrimidin eine bekannte Verbindung (vgl. Chem. Ber. 100, Seiten 591-604).

Die Verbindungen der Formel (IV) sind ebenfalls auf dem Gebiet der organischen Chemie wohlbekannte Verbindungen.

Bei der praktischen Durchführung des Verfahrens b) können geeignete Verdünnungsmittel die vorstehenden inerten organischen Lösungsmittel sein, die beispielhaft für das Verfahren a) angegeben sind, wobei Wasser und Alkohole ausgenommen sind.

Als Basen sind Hydride wie Natriumhydrid und Kaliumhydrid zu nennen.

Das Verfahren b) kann in einem weiten Temperaturbereich, im allgemeinen zwischen etwa 0°C und etwa 100°C, vorzugsweise zwischen etwa 10°C und etwa 50°C, durchgeführt werden.

Die Verbindungen der allgemeinen Formel (I) können auch in Form eines Salzes vorliegen. Beispiele für das Salz sind Salze mit anorganischen Säuren, Salze mit Sulfonsäuren, Salze mit organischen Säuren und Metall-Salze. Demgemäß sind unter den Nitromethylen-tetrahydropyrimidin-Derivaten der Formel (I) in der vorliegenden Erfindung auch deren Salze zu verstehen.

Die aktiven Verbindungen gemäß der vorliegenden Erfindung (I) zeigen starke insektizide Aktivität und können aus diesem Grunde als Insektizide eingesetzt werden. Sie zeigen einen genauen Bekämpfungseffekt gegen Schadinsekten, ohne Schäden bei Kulturpflanzen zu verursachen. Weiterhin können die Verbindungen der vorliegenden Erfindung zur Bekämpfung und Ausrottung eines weiten Bereichs von Schädlingen verwendet werden, darunter saugende Insekten, beißende Insekten und andere Pflanzenparasiten, Schädlinge in Getreidevorräten und gelagerten Körnerfrüchten und gesundheitsgefährdende Schädlinge.

Beispiele für die zu bekämpfenden Schädlinge sind im Folgenden angegeben.

Insekten der Ordnung Coleoptera

Callosobruchus chinensis,

Sitophilus zeamais,

Tribolium castaneum,

Epilachna vigintioctomaculata,

Agriotes fuscicollis,

Anomala rufocuprea,

Leptinotarsa decemlineata,

Diabrotica spp,

Monochamus alternatus,

Lissorhoptus oryzophilus und

Lyctus brunneus;

Insekten der Ordnung Lepidoptera

Lymantria dispar,

Malacosoma neustria,

Pieris rapae,

Spodoptera litura,

Mamestra brassicae,

Chilo suppressalis,

Pyrausta nubilalis,

Ephestia cautella,

Adoxophyes orana,

Carpocapsa pomonella,

Agrotis fucosa,

Galleria mellonella,

Plutella maculipennis und

Phyllocnistis citrella;

Insekten der Ordnung Hemiptera

Nephotettix cincticeps,

Nilaparvata lugens,

Pseudococcus comstocki,

Unaspis yanonensis,

Myzus persicae,

Aphis pomi,

Aphis gossypii

Rhopalosiphum pseudobrassicae,

Stephanitis nashi,

Nazara spp.,

Cimex lectularius,

Trialeurodes vaporariorum und

Psylla spp.;

Insekten der Ordnung Orthoptera

Blatella germanica,

Periplaneta americana,

Gryllotalpa africana und

Locusta migratoria migratoriodes;

Insekten der Ordnung Isoptera

Deucotermes speratus und

Coptotermes formosanus;

Insekten der Ordnung Diptera

Musca domestica,

Aedes aegypti,

Hylemia platura

Culex pipiens,

Anopheles sinensis und

Culex tritaeniorhynchus.

Auf dem Gebiet der Veterinärmedizin sind die neuen Verbindungen gemäß der vorliegenden Erfindung gegenüber verschiedenen schädlichen Tierparasiten (inneren und äußeren Parasiten) wie Insekten und Würmern wirksam.

Spezielle Beispiele für solche Tierparasiten sind nachstehend angegeben.

Insekten

Gastrophilus spp.,

Stomoxys spp.,

Trichodectes spp.,

Rhodnius spp. und

Ctenocephalides canis.

Substanzen mit pestizider Wirkung gegen Schädlinge, darunter sämtliche der oben beispielhaft angegebenen Species, werden in der vorliegenden Anmeldung gelegentlich einfach als "Insektizide" bezeichnet.

Die erfindungsgemäßen Wirkstoffe können in ihren handelsüblichen Formulierungen oder den aus diesen Formulierungen hergestellten Anwendungsformen im Gemisch mit anderen aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die Wirkstoffe gemäß der vorliegenden Erfindung können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren. Die Konzentration der aktiven Verbindung in den Anwendungsformen kann 0.0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger vorzugsweise geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan; Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele

Beispiel 1

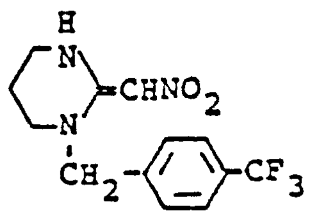

(Verbindung Nr. 1)

N-(4-Trifluormethylbenzyl)trimethylendiamin - (9,28 g) und 1-Nitro-2,2-bis(methylthio)ethylen (6,60 g) wurden in 100 ml Ethanol 8 h unter Rückfluß erhitzt. Die Reaktionsmischung wurde auf Raumtemperatur abgekühlt. Die abgeschiedenen Kristalle wurden durch Filtration gesammelt und mit einer kleinen Menge Ethanol gewaschen, wonach das gewünschte 1-(4-Trifluormethylbenzyl)-2-(nitromethylen)tetrahydropyrimidin (9,6 g) erhalten wurde; Schmp. 147-150°C.

Die nachstehende Tabelle 1 zeigt Verbindungen der vorliegenden Erfindung, die nach einer Arbeitsweise ähnlich der in Beispiel 1 beschriebenen synthetisiert wurden.

Tabelle 1

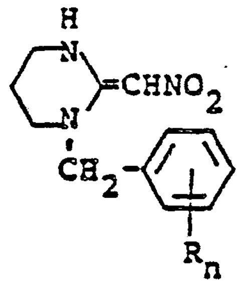

| Verbindung Nr. | $R_n$ | Physikal.Konstante |
|---|---|---|
| 2 | 4-$CH_3$ | Schmp. 180-182°C |
| 3 | 2-$OCH_3$ | Schmp. 198-200°C |
| 4 | 4-$OCH_3$ | Schmp. 140-141°C |
| 5 | 4-$SCH_3$ | Schmp. 173-174.5°C |
| 6 | 3-$NO_2$ | Schmp. 207-209°C |
| 7 | 4-$NO_2$ | Schmp. 178-180°C |
| 8 | 3,4-$Cl_2$ | Schmp. 188-191°C |
| 9 | 3-$N(CH_3)_2$ | Schmp. 210-213°C |
| 10 | 4-$N(CH_3)_2$ | Schmp. 214-216°C |
| 11 | 3-$CF_3$ | Schmp. 188-190°C |
| 12 | 4-$SCF_3$ | Schmp. 197-198°C |

Verbindungen der allgemeinen Formel (I) gemäß der vorliegenden Erfindung, die nach einer ähnlichen Arbeitsweise hergestellt wurden, sind nachstehend aufgeführt: Nr 13:N-(4-Cyanobenzyl)-2-(nitromethylen)tetrahydropyrimidin,

Nr.14: N-(4-Trifluoromethylbenzyl)-2-(nitromethylen)tetrahydropyrimidin,

Nr.15: N-(4-Acetamidobenzyl)-2-(nitromethylen)-tetrahydropyrimidin,

Nr.16: N-(4-Carboxybenzyl)-2-(nitromethylen)-tetrahydropyrimidin,

Nr.17: N-(4-Methoxycarbonylbenzyl)-2-(nitromethylen)tetrahydropyrimidin,

Nr.18: N-(4-Methylsulfinylbenzyl)-2-(nitromethylen)-tetrahydropyrimidin,

Nr.19: N-(4-methylsulfonylbenzyl)-2-(nitromethylen)tetrahydropyrimidin,

Nr.20: N-(3-Aminobenzyl)-2-(nitromethylen)-tetrahydropyrimidin,

Nr.21: N-(4-ethylbenzyl)-2-(nitromethylen)-tetrahydropyrimidin,

Nr.22: N-(4-Aminobenzyl)-2-(nitromethylen)-tetrahydropyrimidin,

Nr.23: N-(3-Cyanobenzyl)-2-(nitromethylen)-tetrahydropyrimidin,

Nr.24: N-(3-Acetamidobenzyl)-2-(nitromethylen)-tetrahydropyrimidin,

Nr.25: N-[4-(1,1,2,2-Tetrafluoroethoxy)benzyl]-2-(nitromethylen)tetrahydropyrimidin,

Nr.26: N-(4-Difluoromethoxybenzyl)-2-(nitromethylen)tetrahydropyrimidin,

Nr.27: N-(4-Ethoxybenzyl)-2-(nitromethylen)-tetrahydropyrimidin,

Nr. 28: N-[4-(2,2,2-Trifluorethylthio)benzyl]-2-(nitromethylen) tetrahydropyrimidin,

Nr. 29: N-(3-Methylbenzyl)-2-(nitromethylen)-tetrahydropyrimidin,

Nr. 30: N-(4-Fluormethylbenzyl)-2-(nitromethylen)-tetrahydropyrimidin) und

Nr. 31: N-/ 4-(2,2,2-Trifluorethoxy)benzyl]-2-(nitromethylen)tetrahydropyrimidin).

Beispiel 2

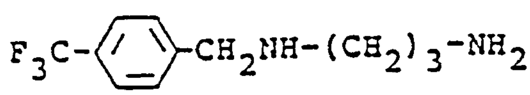

(Verbindung Nr. II-1)

Eine Lösung von 4-Trifluormethylbenzylchlorid (38,9 g) in 50 ml Acetonitril wurde zu einer Lösung von Trimethylendiamin (74 g) in 200 ml Acetonitril unter Rühren bei 5°C bis 15°C hinzugefügt. Nach der Zugabe wurde die Mischung 1 h bei 25°C gerührt. Das überschüssige Trimethylendiamin und Acetonitril wurde unter vermindertem Druck abgedampft. Wasser wurde dem Rückstand zugegeben, und die wäßrige Schicht wurde mit Dichloromethan extrahiert. Das Dichlormethan wurde abgedampft, und der Extrakt wurde im Vakuum destilliert, wonach farbloses N-(4-Trifluormethylbenzyl)-trimethylendiamin (34,8 g) erhalten wurde; Sdp. 134-136°C/1,6 mbar (1,2 mmHg).

Typische Beispiele von Zwischenprodukt-Verbindungen der allgemeinen Formel (II), die nach einer Arbeitsweise ähnlich der in Beispiel 2 beschriebenen erhalten wurden, sind in Tabelle 2 aufgeführt.

## Tabelle 2

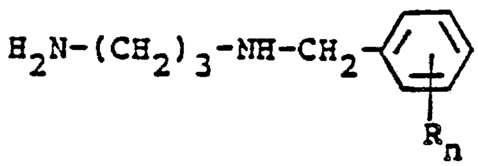

| Verbindung Nr. | $R_n$ | |
|---|---|---|
| II-2 | 4-$CH_3$ | Sdp. 125-130°C/ 1,33 mbar |
| II-3 | 4-$OCH_3$ | Sdp. 144-147°C/ 2,27 mbar |
| II-4 | 2-$OCH_3$ | Sdp. 139-140°C/ 2,0 mbar |
| II-5 | 4-$SCH_3$ | Sdp. 140-142°C/ 1,33 mbar |
| II-6 | 3-$CF_3$ | Sdp. 130°C/ 1,33 mbar |
| II-7 | 4-$NO_2$ | |
| II-8 | 3-$NH_2$ | |
| II-9 | 3-$N(CH_3)_2$ | $n_D^{25}$ 1.5706 |
| II-10 | 4-$SCF_3$ | $n_D^{25}$ 1.5066 |
| II-11 | 4-CN | $n_D^{15}$ 1.5630 |
| II-12 | 4-$OCF_2CHF_2$ | $n_D^{27}$ 1.4642 |

Biologische Test-Beispiele

Vergleichs-Verbindung A-1:

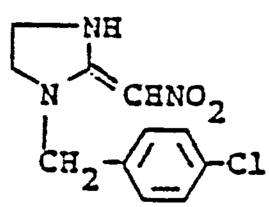

(die in der DE-OS 27 32 660 beschriebene Verbindung).

Beispiel 3

Test mit gegen Organophosphor-Mittel resistenten Nephotettix cincticeps:

Herstellung eines Test-Präparats:

| | | |
|---|---|---|
| Lösungsmittel: | 3 Gew.-Teile | Xylol |
| Emulgator: | 1 Gew.-Teil | Polyoxyethylen-alkylphenylether |

Zur Herstellung eines geeigneten Test-Präparats wurde 1 Gew.-Teil der aktiven Verbindung mit der oben bezeichneten Menge Lösungsmittel, das die oben angegebene Menge Emulgator enthielt, vermischt, und die Mischung wurde mit Wasser auf eine vorher festgesetzte Konzentration verdünnt.

Test-Verfahren:

Auf Reispflanzen von etwa 10 cm Höhe, die jeweils in Töpfe von 12 cm Durchmesser gepflanzt waren, wurden pro Topf 10 ml der mit Wasser verdünnten, eine vorbestimmte Konzentration jeder der aktiven Verbindungen aufweisen den Lösungen die wie oben angegeben hergestellt wurden, gesprüht. Die aufgesprühte Chemikalie wurde trocknen gelassen, und über die Reispflanzen wurde ein Drahtkorb von 7 cm Durchmesser und 14 cm Höhe gestülpt, unter dem 30 ausgewachsene weibliche Exemplare von Nephotettix cincticeps, die gegen Organophosphor-Insektizide resistent waren, ausgesetzt wurden. Die Töpfe wurden jeweils in einem Raum mit konstanter Temperatur aufbewahrt, und 2 Tage später wurde die Zahl der toten Insekten bestimmt und das Vernichtungsverhältnis berechnet.

In diesem Test zeigte beispielsweise die nachstehend bezeichnete Verbindung, Verbindung Nr. 1, eine überlegene Wirkung im Vergleich zum Stand der Technik.

Beispiel 4

Test mit gegen Organophosphor-und Carbamat-Chemikalien resistenten Myzodes persicae (grünen Pfirsichblattläusen):

Test-Verfahren:

Gezüchtete grüne Pfirsichblattläuse, die gegen Organophosphor-Chemikalien und Carbamat-Chemikalien resistent waren, wurden auf Setzlingen von Eierfrüchten (schwarzen länglichen Auberginen) von etwa 20 cm Höhe ausgesetzt, die in unglasierten Töpfen mit einem Durchmesser von 15 cm gezogen worden waren (etwa 200 Blattläuse pro Setzling). Einen Tag nach dem Aussetzen wurde eine wie in Beispiel 3 hergestellte wäßrige Verdünnung jeder der aktiven Verbindungen mit einer vorher festgelegten Konzentration in genügender Menge mit Hilfe einer Spritzpistole auf die Pflanzen aufgesprüht. Nach dem Sprühen wurden die Töpfe in einem Gewächshaus bei 28°C stehen gelassen. 24 Stunden nach dem Sprühen wurde das Vernichtungsverhältnis berechnet. Für jede Verbindung wurde der Test als Doppelbestimmung durchgeführt.

In diesem Test zeigten beispielsweise die nachstehend bezeichneten Verbindungen, Verbindung Nr. 1 und Verbindung Nr.7, eine überlegene Wirkung im Vergleich zum Stand der Technik.

**Ansprüche**

1. Nitromethylen-tetrahydropyrimidine der Formel - (I)

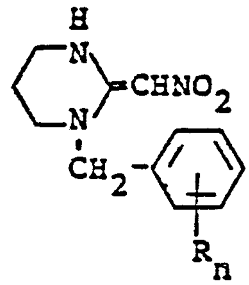

(I)

in der

R eine Alkyl-Gruppe, eine Alkoxy-Gruppe, eine Alkylthio-Gruppe, eine Nitro-Gruppe, eine Cyano-Gruppe, eine halogen-substituierte Alkyl-Gruppe, eine halogen-substituierte Alkoxy-Gruppe, eine halogen-substituierte Alkylthio-Gruppe, eine Amino-Gruppe, eine Dialkylamino-Gruppe, eine Acylamino-Gruppe, eine Carboxyl-Gruppe, eine Alkoxycarbonyl-Gruppe, eine Alkylsulfinyl-Gruppe, eine Alkylsulfonyl-Gruppe oder ein Halogen-Atom bezeichnet und

n 1, 2 oder 3 bezeichnet, jedoch mit der Maßgabe, daß, wenn n für 1 steht, R nicht ein Halogen-Atom bezeichnen darf.

2. Verbindungen nach Anspruch 1, dadurch gekennzeichnet, daß

R eine Alkyl-Gruppe mit 1 bis 4 Kohlenstoff-Atomen,

eine Alkoxy-Gruppe mit 1 bis 4 Kohlenstoff-Atomen, eine Alkylthio-Gruppe mit 1 bis 4 Kohlenstoff-Atomen, eine Nitro-Gruppe, eine Cyano-Gruppe, eine halogen-substituierte Alkyl-Gruppe mit 1 bis 4 Kohlenstoff-Atomen, eine halogen-substituierte Alkoxy-Gruppe mit 1 bis 4 Kohlenstoff-Atomen, eine halogen-substituierte Alkylthio-Gruppe mit 1 bis 4 Kohlenstoff-Atomen, eine Amino-Gruppe, eine Dialkylamino-Gruppe mit 1 bis 4 Kohlenstoff-Atomen in jeder Alkyl-Struktureinheit, eine Acetylamino-Gruppe, eine Carboxyl-Gruppe, eine Alkoxycarbonyl-Gruppe mit 2 bis 5 Kohlenstoff-Atomen, eine Alkylsulfinyl-Gruppe mit 1 bis 4 Kohlenstoff-Atomen oder ein Halogen-Atom ist, und

n 1 oder 2 ist, jedoch mit der Maßgabe, daß, wenn 1 ist, R nicht ein Halogen-Atom bezeichnen darf.

3. Verbindungen nach Anspruch 1, dadurch gekennzeichnet, daß

R Methyl, Ethyl, Methoxy, Ethoxy, Methylthio, Ethylthio, Nitro, Cyano, Trifluormethyl, Trifluormethoxy, Trifluormethylthio, 2,2,2-Trifluorethylthio, Amino, Dimethylamino, Acetylamino, Carboxy, Methoxycarbonyl, Methylsulfinyl, Methylsulfonyl, Fluor , Chlor oder Brom bezeichnet und

n 1 oder 2 ist, jedoch mit der Maßgabe, daß, wenn n 1 ist, R nicht Fluor , Chlor oder Brom bezeichnen darf.

4. Verbindungen nach Ansprüchen 1 bis 3, ausgewählt aus 1-(4-Trifluoromethylbenzyl)-2-(nitromethylen)tetrahydropyrimidin der Formel

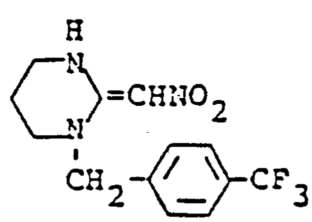

und

1-(4-Nitrobenzyl)-2-(nitromethylen)-tetrahydropyrimidin der Formel

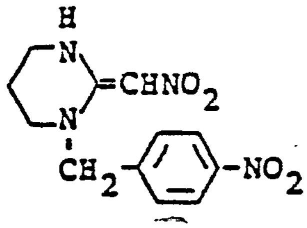

5. Verfahren zur Herstellung von Nitromethylentetrahydropyrimidinen der Formel

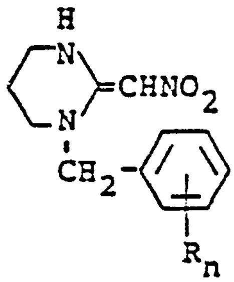

(I)

in der

R eine Alkyl-Gruppe, eine Alkoxy-Gruppe, eine Alkylthio-Gruppe, eine Nitro-Gruppe, eine Cyano-Gruppe, eine halogen-substituierte Alkyl-Gruppe, eine halogen-substituierte Alkoxy-Gruppe, eine halogen-substituierte Alkylthio-Gruppe, eine Amino-Gruppe, eine Dialkylamino-Gruppe, eine Acylamino-Gruppe, eine Carboxyl-Gruppe, eine Alkoxycarbonyl-Gruppe, eine Alkylsulfinyl-Gruppe, eine Alkylsulfonyl-Gruppe oder ein Halogen-Atom bezeichnet und

n 1, 2 oder 3 bezeichnet, jedoch mit der Maßgabe, daß, wenn n für 1 steht, R nicht ein Halogen-Atom bezeichnen darf,

dadurch gekennzeichnet, daß

(a) die Verbindungen der Formel (II)

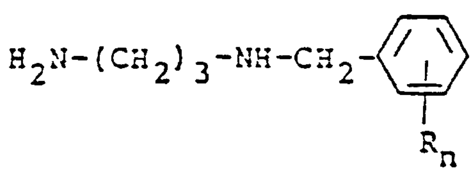

(II)

in der R und n die oben angegebenen Bedeutungen haben, mit Verbindungen der Formel

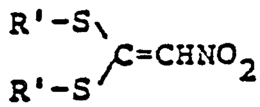

(III)

in der R' eine Niederalkyl-Gruppe oder eine Benzyl-Gruppe bezeichnet, oder die beiden Gruppen R' zusammen genommen eine niedere Alkylen-Gruppe mit wenigstens 2 Kohlenstoff-Atomen bezeichnen oder zusammen mit den benachbarten Schwefel-Atomen einen Ring bilden können, gegebenenfalls in Gegenwart inerter Lösungsmittel umgesetzt werden, oder

(b) 2-Nitromethylen-tetrahydropyrimidin mit Verbindungen der Formel (IV)

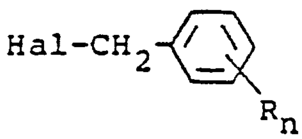

(IV)

in der R und n die im Vorstehenden angegebenen Bedeutungen haben und Hal ein Halogen-Atom ist, umgesetzt wird, gebenenfalls in Gegenwart inerter Lösungsmittel und oder säurebindender Mittel.

6. Insektizide Mittel, dadurch gekennzeichnet, daß sie wenigstens eines der Nitromethylen-tetrahydropyrimidine der Formel (I) nach Ansprüchen 1 bis 5 enthalten.

7. Verfahren zur Bekämpfung von Schadinsekten, dadurch gekennzeichnet, daß man Nitromethylen-tetrahydropyrimidine der Formel (I) nach Ansprüchen 1 bis 5 auf die Insekten und/oder deren Lebensraum einwirken läßt.

8. Verwendung von Nitromethylen-tetrahydropyrimidinen der Formel (I) nach Ansprüchen 1 bis 5 zur Bekämpfung von Schadinsekten.

9. Verfahren zur Herstellung von insektiziden Mitteln, dadurch gekennzeichnet, daß Nitromethylen-tetrahydropyrimidine der Formel (I) nach Ansprüchen 1 bis 5 mit Streckmitteln und/oder grenzflächenaktiven Mitteln vermischt werden.